# EUROPEAN PATENT SPECIFICATION

(11) **EP 3 946 232 B1**
(45) Date of publication and mention of the grant of the patent: **21.10.2026**
(21) Application number: 20713915.5
(22) Date of filing: 31.03.2020
(51) Int. Cl.: A61K 8/34, A61K 8/85, A61Q 5/02, A61Q 5/12

(54) **COSMETIC COMPOSITION COMPRISING SPECIFIC HYPERBRANCHED COPOLYMERS AND ANTIMICROBIAL AGENT**
KOSMETISCHE ZUSAMMENSETZUNG MIT SPEZIFISCHEN HYPERVERZWEIGTEN COPOLYMEREN UND ANTIMIKROBIELLEM MITTEL
COMPOSITION COSMÉTIQUE COMPRENANT DES COPOLYMÈRES HYPERRAMIFIÉS SPÉCIFIQUES ET UN AGENT ANTIMICROBIEN

(30) Priority: 01.04.2019 EP 19166541
(43) Date of publication of application: 09.02.2022
(73) Proprietor: DSM IP Assets B.V., 6411 TE Heerlen (NL)
(72) Inventor: MENDROK-EDINGER, Christine, 4303 Kaiseraugst (CH); HECKER, Karina, 4303 Kaiseraugst (CH)
(74) Representative: dsm-firmenich IP
(86) International application number: PCT/EP2020/059100
(87) International publication number: WO 2020/201272

(56) References cited:
- US-A1- 2015 017 218
- US-A1- 2015 216 789
- US-A1- 2016 100 574
- "Research disclosure", RESEARCH DISCLOSURE, KENNETH MASON PUBLICATIONS, HAMPSHIRE, UK, GB, vol. 606, no. 33, 1 October 2014 (2014-10-01), pages 5, XP007143477, ISSN: 0374-4353, [retrieved on 20140916]
- KUNLAYAKORN LAWAN ET AL: "Antimicrobial efficacy of caprylyl glycol and ethylhexylglycerine in emulsion", vol. 23, no. 1, 1 January 2009 (2009-01-01), pages 1 - 3, XP008160700, ISSN: 0857-4421, Retrieved from the Internet <URL:http://www.jhr.cphs.chula.ac.th/abstract.php?abname=Antimicrobial%20Efficacy%20of%20Caprylyl%20Glycol%20and%20Ethylhexylglycerinein%20Emulsion&id=250>
- YAN XUE ET AL: "Antimicrobial Polymeric Materials with Quaternary Ammonium and Phosphonium Salts", INTERNATIONAL JOURNAL OF MOLECULAR SCIENCES, vol. 16, no. 2, 6 February 2015 (2015-02-06), pages 3626 - 3655, XP055493340, DOI: 10.3390/ijms16023626
- HADI BAKHSHI ET AL: "Hyperbranched polyesters as biodegradable and antibacterial additives", JOURNAL OF MATERIALS CHEMISTRY B, vol. 5, no. 33, 1 January 2017 (2017-01-01), GB, pages 6827 - 6834, XP055587856, ISSN: 2050-750X, DOI: 10.1039/C7TB01301A

## Description

### Technical Field

The present invention relates to the field of cosmetic compositions, particularly to the field antimicrobial agents.

### Background of the invention

Antimicrobial agents are known ingredients to stabilizes cosmetic compositions, by reducing the growth of microorganisms. The reduction of growth of microorganisms is essential for the consumers safety and health when using a cosmetic product. By reducing the growth of microorganisms, the shelf life of a cosmetic product can be increased. As the use of certain antimicrobial agents are under public discussions a reduction of antimicrobial agents is a strong desire in the cosmetic industry.

EP 2794729 B1 disclose the preparation of specific hyperbranched polyesters which can be used as flocculants in paper production and in dishwater detergents.

EP 2296619 B1 discloses that specific hyperbranched copolymers have an advantageous effect in a shampoo for increasing the volume of hair.

US 2015/216789 describes a specific quaternized hyperbranched polymer having end-groups of a specific formula (I) characterized in that said quaternized hyperbranched polymer is obtainable by preparation of a hyperbranched polymer having dimethylamino end groups by condensation of 2-dodecen-1-ylsuccinic anhydride, diisopropanolamine and N,N-bis[3-(dimethylamino)propyl]amine followed by quaternization of the dimethylamino end-groups to end groups of formula (I).

The Research disclosure published by KENNETH MASON PUBLICATIONS, HAMPSHIRE, UK, GB, vol. 606, no. 33, 1 October 2014, page 5, describes a formulation comprising polyquaternium-110.

US 2016/100574 describes an antimicrobial agent comprising at least one glycerylether of a specific formula (I).

The article of Kunlayakorn Lawan et al., titled "Antimicrobial efficacy of caprylyl glycol and ethylhexylglycerine in emulsion", published in the Journal of Health Research, College of Public Health Sciences, Chulalongkorn University, Thailand, vol. 23, No. 11, January 2009, pages 1-3, describes antimicrobial efficacies of caprylyl glycol and ethylhexylglycerin in an emulsion.

US 2015/017218 A1 describes a cleansing composition including a preservative enhancing agent selected from the group consisting of caprylyl glycol; caprylyl glyceryl ether; glyceryl caprylate; sorbitan caprylate; ethylhyexyl glycerin, and combinations thereof.

### Summary of the invention

Therefore, the problem to be solved by the present invention is to offer a cosmetic composition with enhanced antimicrobial properties.

It has been surprisingly found that the cosmetic composition of claim 1 is able to solve this problem. It has been particularly observed that a specific hyperbranched copolymer is able to enhance the antimicrobial action of antimicrobial agents used in cosmetic compositions. This effect is a synergistic effect of the specific hyperbranched copolymer on to the antimicrobial effect of an antimicrobial agent of the cosmetic composition. This surprising finding is very advantageous as this invention can lead to higher stability, longer shelf life, respectively, to the reduction of antimicrobial agents in cosmetic compositions by, nevertheless, maintaining the antimicrobial properties.

Further aspects of the invention are subject of further independent claims. Particularly preferred embodiments are subject of dependent claims.

### Detailed description of the invention

In a first aspect the present invention relates to a cosmetic composition comprising
a) a hyperbranched copolymer **(HBC)** of the monomers
   (i) dodecenyl succinic acid anhydride
   (ii) diisopropanol amine
   (iii) bis-dimethylaminopropyl amine
      having terminal groups of the formula 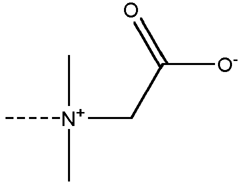
      and having a molecular weight Mn of between 1200 and 4000 g/mol; and
b) an antimicrobial agent of the formula (I) 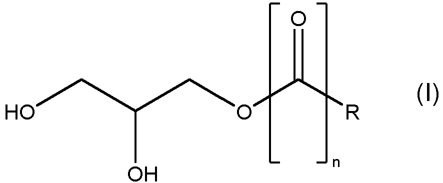
   wherein R is a C₅-C₁₀ alkyl group or C₅-C₁₀ cyclo alkyl group, preferably a C₆-C₉ alkyl group or a C₆-C₉ cyclo alkyl group, more preferably a C₈ alkyl group or a C₆ cyclo alkyl group;
   and wherein n is 0.

The term "molecular weight Mn" stands for the number average molecular weight.

The term "preparation" or "formulation" is used in this document as equivalent to the term "composition".

The term "antimicrobial agent" is used in this document for an organic chemical substance which reduces the growth of microorganisms. However, monohydroxy-C₁-C₆ alkanes, such as methanol, ethanol, propanol or iso-propanol, for this invention, are not considered to be antimicrobial agents. For sake of clarity, it is worthwhile that antioxidants are not to be regarded as antimicrobial agents.

The hyperbranched copolymer **(HBC)** is preferably prepared by the following consecutive steps of:
a1) polymerizing the monomers (i) and monomers (ii) and monomers (iii) to yield a polyesteramide having terminal dimethyl amino groups of the formula 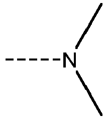
a2) quaternization of the dimethyl amino groups of the polyesteramide of step a1) by 2-chloroacetate, particularly by sodium 2-chloroacetate.

Details for the polymerization step a1) to yield the respective polyesteramide having terminal dimethyl amino groups of the formula 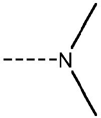 are disclosed for example by EP 2 794 729 B1.

Preferably in the polymerization step a1) the monomer (iii) is added to a mixture of monomers (ii) and (iii) under stirring, followed by heating.

Details of the quaternization step a2) are disclosed as well by EP 2 794 729 B1.

The amount of the hyperbranched copolymer (HBC)of the monomers
(i) dodecenyl succinic acid anhydride
(ii) diisopropanol amine
(iii) bis-dimethylaminopropyl amine
   having terminal groups of the formula 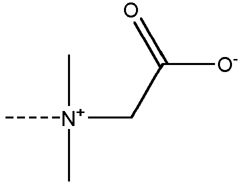
   and having a molecular weight Mn of between 1200 and 4000 g/mol
   in the cosmetic composition is typically between 0.005 and.5.0 % by weight, preferably between 0.05 and 5.0 % by weight, more preferably between 0.1 and 3.0 % by weight, most preferably between 0.5 and 2.5 % by weight, based on the total weight of the cosmetic composition.

It is preferred that the molar ratio of the monomers (i) to monomers (ii) is between 5: 1 and 0.5 : 1, particularly between 4 : 1 and 1 : 1, preferably between 3 : 1 and 3 : 2.

It is further preferred that the molar ratio of the monomers (i) to monomers (iii) is between 5 : 1 and 0.5 : 1, particularly between 3 : 1 and 1 : 1, preferably between 2.5 : 1 and 1.1 : 1.

The hyperbranched copolymer **(HBC)** has preferably a number average molecular weight Mₙ of between 1400 and 3000 g/mol, preferably between 2100 and 2400 g/mol.

Preferably, the hyperbranched copolymer **(HBC)** is polyquaternium-110, also identified by CAS Number 1323977-82-7.

The cosmetic composition comprises an antimicrobial agent of the formula (I) 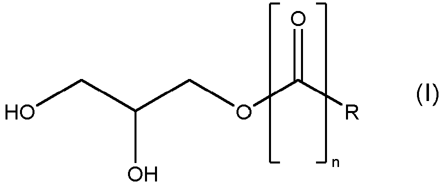
wherein R is a C₅-C₁₀ alkyl group or C₅-C₁₀ cyclo alkyl group, preferably a C₆-C₉ alkyl group or a C₆-C₉ cyclo alkyl group, more preferably a C₈ alkyl group or a C₆ cyclo alkyl group;
and wherein n is 0.

R is preferably cyclohexyl or n-octyl or a branched octyl group, preferably an ethylhexyl group, more preferably a 2-ethylhexyl group.

The antimicrobial agent of the formula (I) is preferably selected from the group consisting of cyclohexyl glycerin, hexyl glycerine, ethylhexyl glycerine, preferably ethylhexyl glycerine and hexyl glycerine. In the most preferred embodiment the antimicrobial agent of the formula (I) is 3-(2-ethylhexyloxy)-1,2-propandiol.

It is possible that the cosmetic composition comprises more than one antimicrobial agent. For example, the cosmetic composition may comprise two or more different antimicrobial agents of formula (I), resulting from different choices of R in formula (I). For example, the cosmetic composition may also comprise additionally to antimicrobial agent(s) of formula (I) any further antimicrobial agent(s) of a formula, which is/are different from formula (I).

It is preferred that the amount of the antimicrobial agent is 0.01 - 6.0 % by weight, preferably 0.05 - 5.0 % by weight, more preferably 0.1 - 3.0 % by weight, based on the total weight of the cosmetic composition.

The weight ratio of said hyperbranched copolymer **(HBC)** and antimicrobial agent is preferably in the range of between 1:30 and 30:1, more preferred between 1:20 - 20:1, even more preferred between 1:10 and 10:1.

In a preferred embodiment the weight ratio of said hyperbranched copolymer **(HBC)** and antimicrobial agent is larger than 1.

The cosmetic composition further preferably comprises at least one emulsifier, preferably an anionic emulsifier. Preferably the anionic emulsifier is an anionic emulsifier selected from the group consisting of potassium cetyl phosphate, disodium cetearyl sulfosuccinate, sodium stearoyl glutamate, sodium stearoyl lactylate, glyceryl stearate citrate and sodium cocoyl isethionate.

Potassium cetyl phosphate is commercially available as Amphisol^{®} K at DSM Nutritional Products Ltd Kaiseraugst.

The amount of emulsifier is preferably in the range between 0.1 - 6.0 % by weight, more preferably between 0.25 - 5.0 % by weight, particularly between 0.5 - 4.0 % by weight, based on the total weight of the cosmetic composition.

The composition is preferably sulfate-free.

Hence, the cosmetic composition is preferably particularly free of sulfates of the group consisting of alkyl sulfates, alkyl ether sulfates, alkyl amido ether sulfates, alkylaryl polyether sulfates and monoglycerides sulfate as well as mixtures thereof.

The term "free" as used in the present document, for example in "sulfate-free", is used to mean that the respective substance is only present at amounts of less than 0.5 % by weight, particularly less than 0.1 % by weight, more particularly below 0.05 % by weight, relative to the weight of the composition. Preferably, "free" means that the respective substance is completely absent in the composition.

The term "sulfate-free" is used in the present document to mean that the composition is free of any anionic tenside having a terminal anionic group of the formula

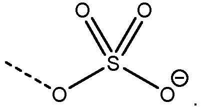

The cosmetic composition is preferably free of cationic emulsifiers. Typical example for such cationic emulsifiers are isostearamidopropyl dimethylamine, stearalkonium chloride, stearamidoethyl diethylamine, behentrimonium methosulfate, behenoyl PG-trimonium chloride, cetrimonium bromide, behenamidopropyl dimethylamine behenate, brassicamidopropyl dimethylamine, stearamidopropyl dimethylamine stearate, cocamidopropyl PG-dimonium chloride, distearoylethyl hydroxyethylmonium methosulfate, dicocoylethyl hydroxyethylmonium methosulfate, distearoylethyl dimonium chloride, shea butteramidopropyltrimonium chloride, behenamidopropyl dimethylamine, brassicyl isoleucinate esylate, acrylamidopropyltrimonium chloride/acrylates copolymer, linoleamidopropyl ethyldimonium ethosulfate, dimethyl lauramine isostearate, isostearamidopropyl laurylacetodimonium chloride, particularly behentrimonium chloride, distearyldi-monium chloride, cetrimonium chloride, steartrimonium chloride, and palmitamido-propyltrimonium chloride.

The cosmetic composition further may comprise cosmetic carriers, excipients and diluents as well as additives and active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

Such possible ingredients of the cosmetic composition are particularly enhance the performance and/or consumer acceptability such as preservatives, antioxidants, fatty substances/oils, thickeners, softeners, light-screening agents, moisturizers, fragrances, co-surfactants, fillers, sequestering agents, cationic-, nonionic- or amphoteric polymers or mixtures thereof, acidifying or basifying agents, viscosity modifiers, and natural hair nutrients such as botanicals, fruit extracts, sugar derivatives and/or amino acids or any other ingredients usually formulated into cosmetic compositions. The necessary amounts of the adjuvants and additives can, based on the desired product, easily be chosen by a person skilled in the art in this field and will be illustrated in the examples, without being limited hereto.

The additional ingredients can either be added to the oily phase, the aqueous phase or separately as deemed appropriate.

In an advantageous embodiment, the compositions according to the present invention comprise from 50% to 99%, preferably from 60% to 98%, more preferably from 70% to 98%, such as in particular from 80% to 95% of a carrier, based on the total weight of the cosmetic composition.

In a particular advantageous embodiment, the carrier consists furthermore of at least 40 wt. %, more preferably of at least 50 wt.-%, most preferably of at least 55 wt.-% of water, such as in particular of 55 to 90 wt.-% of water.

Furthermore, it is preferred that the cosmetic composition comprises at least one UV filter. The UV filter may be a liquid or solid UV filter. The UV filter may be a UV-A or UV-B filters.

Suitable liquid UV-filter absorb light in the UVB (280 - 315 nm) and/ or UVA (315 - 400 nm) range and are liquid at ambient temperature (i.e. 25°C). Such liquid UV-filter are well known to a person in the art and encompass in particular cinnamates such as e.g. octyl methoxycinnamate (PARSOL^{®} MCX) and isoamyl methoxycinnamate (Neo Heliopan^{®} E 1000), salicylates such as e.g. homosalate (3,3,5 trimethylcyclohexyl 2-hydroxybenzoate, PARSOL^{®} HMS) and ethylhexyl salicylate (also known as ethylhexyl salicylate, 2 ethylhexyl 2-hydroxybenzoate, PARSOL^{®} EHS), acrylates such as e.g. octocrylene (2 ethylhexyl 2-cyano-3,3-diphenylacrylate, PARSOL^{®} 340) and ethyl 2-cyano-3,3 diphenylacrylate, esters of benzalmalonic acid such as in particular dialkyl benzalmalonates such as e.g. di (2-ethylhexyl) 4-methoxybenzalmalonate and polysilicone 15 (PARSOL^{®} SLX), dialkylester of naphthalates such as e.g. diethylhexyl 2,6-naphthalate (Corapan^{®} TQ), syringylidene malonates such as e.g. diethylhexyl syringylidene malonate (Oxynex^{®} ST liquid) as well as benzotriazolyl dodecyl p-cresol (Tinoguard^{®} TL) as well as benzophenone-3 and drometrizole trisiloxane.

Particular advantageous liquid UV-filter are octyl methoxycinnamate, homosalate, ethylhexyl salicylate, octocrylene, diethylhexyl 2,6-naphthalate, diethylhexyl syringylidene malonate, benzotriazolyl dodecyl p-cresol, benzophenone-3, drometrizole trisiloxane, Polysilicone-15 as well as mixtures thereof.

Suitable solid UV-filter absorb light in the UVB and/ or UVA range and are solid at ambient temperature (i.e. 25°C). They are particularly solid organic UV filters. Particularly suited solid UV-filters are of the group consisting of bis-ethyl-hexyloxyphenol methoxyphenyl triazine, butyl methoxydibenzoyl methane, methylene bis-benzotriazolyl tetramethylbutylphenol, diethylamino hydroxybenzoyl hexyl benzoate, ethylhexyl triazone, diethylhexyl butamido triazone and 4-methylbenzylidene camphor.

The amount of an individual organic UV filter is preferably in the range of 0.1 to about 6 % by weight, preferable in the range of 0.5 to 5 % by weight, most preferably in the range of 1 to 4 % by weight, based on the total weight of the cosmetic composition.

In case of a sun care composition, the total amount of organic UV filter (s) depends strongly on the targeted UV protection of said composition and is typically in the range of between 1 to 50% by weight, preferably between 5 to 40% by weight, based on the total weight of said composition.

A sun creme with an SPF 15 (SPF=sun protection factor), for example, comprises preferably a total amount of organic UV filter (s) of between 4 to 20% by weight, more preferably between 7 and 15 % by weight, based on the total weight of said composition.

A sun creme with an SPF 30, for example, comprises preferably a total amount of organic UV filter (s) of between 10 to 40% by weight, more preferably between 15 and 25 % by weight, based on the total weight of said composition.

A sun creme with an SPF 50, for example, comprises preferably a total amount of organic UV filter (s) of between 15 to 50% by weight, more preferably between 20 and 40 % by weight, based on the total weight of said composition.

Particularly suitable thickeners in all embodiments of the present invention are xanthan gum, gellan gum and/ or carboxymethylcellulose. Most preferably in all embodiments of the present invention the thickener is xanthan gum or gellan gum. Further suitable thickeners are polyacrylates such as commercially available under the trade name Carbomer, or acrylate/C10-30 alkyl acrylate crosspolymers or salts of polyacrylic acid or polyacrylamides.

Such thickener(s) are preferably used in an amount (total) selected in the range from 0.1 to 1 wt.-%, more preferably in an amount of 0.1 to 0.5 wt.-%, based on the total weight of the cosmetic composition.

It is preferred that the composition is free of polyvinylpyrrolidones (PVP), particularly free of alkylated polyvinylpyrrolidiones, such as copolymers of N-vinyl-pyrrolidones and hexadecane or eicosene, e.g. as commercially available as Antaron V-216 or Antaron V-220.

The cosmetic compositions according to the invention in general have a pH in the range from 3 to 10, preferably a pH in the range from 4 to 8 and most preferably a pH in the range from 4 to 7.5. The pH is adjusted by methods known to a person skilled in the art, e.g. by using an acid such as a hydroxy acid including glycolic acid, lactic acid, malic acid, citric acid and tartaric acid or a base such as e.g. sodium or potassium hydroxide or ammonium hydroxide as well as mixtures thereof.

Preferably, in the compositions according to the invention citric acid in an amount of at least 0.0001 wt.-%, such as e.g. in an amount of 0.01-1 wt.-%, in particular in an amount of 0.01 to 0.5 wt.-% is used for pH adjustment.

The cosmetic composition is preferably sulfate-free and/or free of para-bens, and/or silicon oils and/or silicone surfactants and/or methylisothiazolidine and/or free of polyvinylpyrrolidones (PVP), particularly free of alkylated polyvinylpyrrolidiones.

The cosmetic composition is preferably a topical composition.

The term "topical" as used herein is understood here to mean external application to keratinous substances, which are in particular the skin, scalp, eyelashes, eyebrows, nails, mucous membranes and hair, preferably the skin.

As the topical compositions are intended for topical application, it is well understood that they comprise a physiologically acceptable medium, i.e. a medium compatible with keratinous substances, such as the skin, mucous membranes, and keratinous fibres. In particular, the physiologically acceptable medium is a cosmetically acceptable carrier.

The term "cosmetically acceptable carrier" refers to all carriers and/or excipients and/ or diluents conventionally used in cosmetic compositions such as in particular in sun care products.

Preferably the cosmetic composition is a skin care composition.

In a further embodiment, the cosmetic composition is a decorative preparation or a functional preparation.

Examples of skin care compositions are, in particular, light protective composition, anti-ageing composition, compositions/preparations for the treatment of photo-ageing, body oils, body lotions, body gels, treatment creams, skin protection ointments, skin powders, moisturizing gels, moisturizing sprays, face and/or body moisturizers, skin-tanning preparations (i.e. compositions for the artificial/sunless tanning and/or browning of human skin), for example self-tanning creams as well as skin lightening compositions.

Examples of functional preparations are cosmetic compositions containing active ingredients such as hormone preparations, vitamin preparations, vegetable extract preparations, anti-ageing preparations, and/or antimicrobial (antibacterial or antifungal) preparations without being limited thereto.

The cosmetic composition is preferably a skin care composition.

In a particular embodiment, the cosmetic composition is a sun care composition. Sun care compositions are light-protective composition (sun care products), such as sun protection milks, sun protection lotions, sun protection creams, sun protection oils, sun blocks or day care creams with a SPF (sun protection factor). Sun protection creams, sun protection lotions, sun protection milks and sun protection compositions are of particular interest.

The cosmetic compositions according to the present invention may be in the form of a suspension or dispersion in solvents or fatty substances, or alternatively in the form of an emulsion or micro emulsion (in particular of oil-in-water (O/W-) or water-in-oil (W/O-)type, silicone-in-water (Si/W-) or water-in-silicone (W/Si-)type, PIT-emulsion, multiple emulsion (e.g. oil-in-water-in oil (O/W/O-) or water-in-oil-in-water (W/O/W-)type), pickering emulsion, hydrogel, alcoholic gel, lipogel, one- or multiphase solution or vesicular dispersion or other usual forms, which can also be applied by pens, as masks or as sprays.

Preferred cosmetic compositions in all embodiments of the present invention are emulsions which contain an oily phase and an aqueous phase such as in particular O/W, W/O, Si/W, W/Si, O/W/O, W/O/W multiple or a pickering emulsions.

The total amount of the oily phase present in such emulsions is preferably at least 10 wt.-%, such as in the range from 10 to 60 wt.-%, preferably in the range from 15 to 50 wt.-%, most preferably in the range from 15 to 40 wt.-%, based on the total weight of the cosmetic composition.

The amount of the aqueous phase present in such emulsions is preferably at least 20 wt. %, such as in the range from 40 to 90 wt.-%, preferably in the range from 50 to 85 wt.-%, most preferably in the range from 60 to 85 wt.-%, based on the total weight of the cosmetic composition.

The cosmetic compositions can be in the form of a liquid, lotion, a thickened lotion, a gel, a cream, a milk, an ointment or a paste.

More preferably, the cosmetic compositions according to the present invention are in the form of an oil-in-water (O/W) emulsion comprising an oily phase dispersed in an aqueous phase in the presence of an O/W- respectively Si/W-emulsifier. The preparation of such O/W emulsions is well known to a person skilled in the art.

The compositions in form of O/W emulsions according to the invention can be provided, for example, in all the formulation forms for O/W emulsions, for example in the form of serum, milk or cream, and they are prepared according to the usual methods. The compositions which are subject-matters of the invention are preferably intended for topical application and can in particular constitute a dermatological or cosmetic composition, for example intended for protecting human skin against the adverse effects of UV radiation (antiwrinkle, anti-ageing, moisturizing, anti-sun protection and the like).

It is preferred that the cosmetic composition is a shampoo or a hair conditioner.

It is further preferred, that the cosmetic composition is a topical composition applied to the human skin, scalp and/ or hair.

It has been surprisingly found that hyperbranched copolymer **(HBC)** increases the antimicrobial action of antimicrobial agents in cosmetic compositions.

Herein is further described the use of a hyperbranched copolymer **(HBC)** of the monomers
(i) dodecenyl succinic acid anhydride
(ii) diisopropanol amine
(iii) bis-dimethylaminopropyl amine
   having terminal groups of the formula 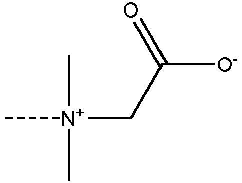
   and having a molecular weight Mn of between 1200 and 4000 g/mol; to enhance the antimicrobial action of an antimicrobial agent in a cosmetic composition.

Antimicrobial action has been observed particularly against the microorganisms which are selected from the group consisting *Kocuria rhizophila, Staphylococcus aureus, Enterobacter gergoviae, Escherichia coli, Klebsiella pneumoniae, Pseudomonas aeruginosa, Pseudomonas fluorescens, Pseudomonas putida, Aspergillus brasiliensis, Penicillium pinophilum and Candida albicans,* particularly against *Escherichia coli.*

The use of a hyperbranched copolymer **(HBC)** to enhance the antimicrobial action of an antimicrobial agent in a cosmetic composition can take place both in the cosmetic sense as well as in the pharmaceutical sense. In all embodiments of the present invention, the use is, however, preferably a cosmetic (i.e. a non-therapeutic) use, such as for maintenance of skin homeostasis and/or balancing the skin microbiome and/or hair microbiome.

It has been observed that, when adding the above hyperbranched copolymer **(HBC)** to cosmetic compositions which comprise an antimicrobial agent, the antimicrobial action is increased significantly. In other words, by adding additionally the hyperbranched copolymer **(HBC)** to a cosmetic composition, which comprises an antimicrobial agent, a smaller number of microbes are observed to grow than without the use of said hyperbranched copolymer **(HBC).** The above hyperbranched copolymer **(HBC)** has a synergistic effect on the antimicrobial action of an antimicrobial agent in a composition, particularly in a cosmetic composition.

This is very advantageous also in such a way that the amount of an existing antimicrobial agents can be reduced by adding the effective amount of the above hyperbranched copolymer **(HBC)** to maintain the same antimicrobial action This is particularly advantageous because certain existing antimicrobial agents are under public discussion and, hence, there exists a great desire in decreasing the amount of such antimicrobial agents.

It has been further observed that the antimicrobial action is particularly pronounced in an aqueous phase. This is very advantageous as it is known that it is typically the water phase of a product which is most susceptible to microbial growth.

In a final aspect, the invention relates to the use of a hyperbranched copolymer (HBC) of the monomers
(i) dodecenyl succinic acid anhydride
(ii) diisopropanol amine
(iii) bis-dimethylaminopropyl amine
   having terminal groups of the formula 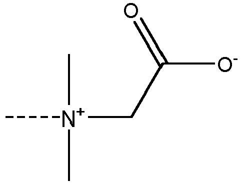
   and having a molecular weight Mn of between 1200 and 4000 g/mol; to enhance synergistically the antimicrobial activity of an antimicrobial agent of the formula (I) 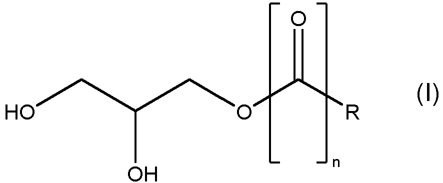
   wherein R is a C₅-C₁₀ alkyl group or C₅-C₁₀ cyclo alkyl group, preferably a C₆-C₉ alkyl group or a C₆-C₉ cyclo alkyl group, more preferably a C₈ alkyl group or a C₆ cyclo alkyl group;
   and wherein n is 0.

The details to the hyperbranched copolymer **(HBC)** and antimicrobial agent have been described above for the use in great details.

### Examples

The present invention is further illustrated by the following experiments.

### Preparation of hyperbranched copolymer (HBC1)

The hyperbranched copolymer ***HBC1*** of the monomers dodecenyl succinic acid anhydride and diisopropanol amine and bis-dimethylaminopropyl amine has been prepared according to example 3 in EP 2 794 729 B1 using 237.59 g of N,N-bis(N'N'-dimethylaminopropyl)amine and 112.6 g diisopropanol amine and 426.89 g of dodecenylsuccinic anhydride. After heating and vacuum, the residual carboxylic acid content of < 0.3 meq/g (tritrimetrical analysis) AV=9.8mg KOH/g and amine content of 2.99 meq/g (tritrimetrical analysis) and a molecular weight Mn=2240 Da was obtained. This product has been reacted with sodium chloroacetate in water and stirred at 80°C until ¹H-NMR analysis shows a complete conversion of the chloroacetate to obtain the hyperbranched copolymer ***HBC1*** which has terminal groups of the formula 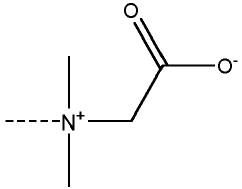 and a molecular weight Mn of 2.3 kDa.

The hyperbranched copolymer ***HBC1*** was used as a 50 % solution in water in the following experiments. The amounts indicated in the tables 1, 2 and 3 are based on the amount of polymer.

### Experimental series 1

### Preparation of cosmetic compositions

Shampoo compositions, to which the above hyperbranched copolymer ***HBC1*** and/or ethylhexylglycerin (=3-(2-ethylhexyloxy)-1,2-propandiol) have been added, have been prepared as indicated in table 1.

### Testing of antimicrobial action

The antimicrobial efficacy is assessed in analogy to the regulatory challenge test method (NF EN ISO11930). In this test the following mixture of bacteria, yeast and molds:

| | |
|---|---|
| -grampositive bacteria : | *Kocuria rhizophila, Staphylococcus aureus* |
| -gramnegative bacteria : | *Enterobacter gergoviae, Escherichia coli, Klebsiella pneumoniae, Pseudomonas aeruginosa, Pseudomonas fluorescens, Pseudomonas putida* |
| -moulds: | *Aspergillus brasiliensis, Penicillium pinophilum* |
| -yeast: | *Candida albicans* |

are inoculated 6 times (once a week) into the test material. Each week before the next inoculation the germs in the test material are counted. The inoculum contains pathogenic microorganisms as germs which are well known for product spoilage. All species have to be cultivated separately and mixed directly before the addition, to ensure a constant composition and germ count of the inoculum. Its germ count is approximately 10⁷⁻⁸ cfu/ml in the sample. After the 6 inoculation cycles the microbial growth has been checked and assessed as follows:

| Growth: | symbol: | assessment of antimicrobial action: |
|---|---|---|
| Free of growth: | - | passed |
| Slight growth: | + | passed |
| Moderate growth: | ++ | failed |
| Heavy growth: | +++ | failed |

The results are compiled in table 1.

**Table 1 Antimicrobial action in shampoo compositions.**

| **Shampoo Compositions** | | | | | |
|---|---|---|---|---|---|
| **Ingredients (INCI) [wt.%]** | ***Ref.1*** | ***Ref.2*** | ***Ref.3*** | ***1*** | ***2*** |
| CI 42090 | 0.001 | 0.001 | 0.001 | 0.001 | 0.001 |
| CI 47005 | 0.001 | 0.001 | 0.001 | 0.001 | 0.001 |
| Acrylates/steareth-20 methacrylate crosspolymer | 5.0 | 5.0 | 5.0 | 5.0 | 5.0 |
| Sodium lauroyl glutamate, aqua, sodium chloride | 3.0 | 3.0 | 3.0 | 3.0 | 3.0 |
| Sodium laureth sulfate, aqua | 35.0 | 35.0 | 35.0 | 35.0 | 35.0 |
| Sodium cocoamphoacetate, aqua, sodium chloride | 3.0 | 3.0 | 3.0 | 3.0 | 3.0 |
| PEG-40 hydrogenated castor oil | 0.5 | 0.5 | 0.5 | 0.5 | 0.5 |
| PEG-7 glyceryl cocoate | 0.5 | 0.5 | 0.5 | 0.5 | 0.5 |
| Parfum | 0.8 | 0.8 | 0.8 | 0.8 | 0.8 |
| Octocrylene, homosalate, butyl methoxydibenzoylmethane, helianthus annuus seed oil | 0.1 | 0.1 | 0.1 | 0.1 | 0.1 |
| Polyquaternium-22, aqua | 1.5 | 1.5 | 1.5 | 1.5 | 1.5 |
| Cocamidopropyl betaine, aqua, sodium chloride | 5.2 | 5.2 | 5.2 | 5.2 | 5.2 |
| Saccharide derivative, aqua, citric acid, sodium citrate | 0.5 | 0.5 | 0.5 | 0.5 | 0.5 |
| Aqua, sodium hydroxide | 0.5 | 0.5 | 0.5 | 0.5 | 0.5 |
| ***HPC1*** | 0.75 | 1.50 | | 0.75 | 1.50 |
| Ethylhexylglycerin | | | 0.60 | 0.60 | 0.60 |
| Aqua | Ad 100 | Ad 100 | Ad 100 | Ad 100 | Ad 100 |
| Microbial growth | ++ | ++ | +++ | - | - |
| Assessment | failed | failed | failed | passed | passed |

The results of table 1 show that the effect that combination of these specific copolymers, i.e. the hyperbranched copolymers, and the antimicrobial agent of the formula (I) show a synergistic effect against a broad variety of microorganisms.

### Experimental series 2

### Preparation of cosmetic compositions

O/W skin care emulsion to which the above hyperbranched copolymer ***HBC1*** and/or n-hexylglycerin have been added, have been prepared as indicated in table 2.

### Testing of antimicrobial action (Challenge test)

The antimicrobial action has further been tested according to ISO 11930 on the O/W skin care emulsion compositions according to table 2 for *Escherichia coli.*

The evaluation of the preservation of a cosmetic formulation is based on inoculation of the formulation with calibrated inocula (prepared from relevant strains of micro-organisms). The number of surviving micro-organisms is measured at defined intervals during a period of 28 days. The number of microorganisms has been counted directly after inoculation and after 7 days of growth and are indicated in table 2.

**Table 2 Antimicrobial action of compositions on Escherichia coli after 7 days. Directly after inoculation the count was 440'000 [cfu/ml].**

| **O/W skin care emulsion compositions** | | | |
|---|---|---|---|
| **Ingredients (INCI) [wt.%]** | ***Ref.4*** | ***Ref.5*** | ***3*** |
| Potassium Cetyl Phosphate | 2.0 | 2.0 | 2.0 |
| Cetearyl Alcohol | 3.0 | 3.0 | 3.0 |
| Cetyl Alcohol | 2.0 | 2.0 | 2.0 |
| Cosmetic oil | 3.0 | 3.0 | 3.0 |
| C12-15 Alkyl Benzoate | 15.0 | 15.0 | 15.0 |
| Xanthan Gum | 0.3 | 0.3 | 0.3 |
| Glycerin | 3.0 | 3.0 | 3.0 |
| ***HPC1*** | 1.5 | | 1.5 |
| n-Hexylglycerin | | 0.3 | 0.3 |
| Aqua | Ad 100 | Ad 100 | Ad 100 |
| Colony counts after 7 days [cfu/ml] | 190'000 | 8'600 | 5'700 |

### Experimental series 3

### Testing of antimicrobial action (24 hours Challenge test)

Since it is known that typically a water phase of a product is most susceptible to microbial growth, the antimicrobial efficacy has been further assessed in analogy to the regulatory challenge test method (NF EN ISO11930) for an aqueous solution.

Thus, a solution of ***HPC1*** and/or n-Hexylglycerin in an amount as indicated in table 3 were prepared under sterile conditions. For this, it was solubilized in physiological serum with 0.85 wt.-% NaCl. The solutions were deposed in 96-deep well plates (1.6 ml/well). The wells were contaminated with *Escherichia coli,* at 3.8*10⁵ to 6.1*10⁵ cfu/ml. After the contamination each well was thoroughly mixed to ensure a homogeneous distribution of the microorganism. Then each plate was incubated at 22°C for 24h. The counting of the (remaining) population was carried out 24h after contamination and reported in table 3.

**Table 3 Antimicrobial action in the 24 hours screen test with E.coli.**

| | ***Ref.6*** | ***Ref.7*** | ***Ref.8*** | **4** |
|---|---|---|---|---|
| ***HPC1*** [wt.%] | | 0.5 | | 0.5 |
| n-Hexylglycerin [wt.%] | | | 0.2 | 0.2 |
| Colony counts after 24h [cfu/ml] | 1'000'000 | <1'000'000 | 1'000'000 | 0 |

### Experimental series 4

### Testing of antimicrobial action (24 hours Challenge test)

Since it is known that typically a water phase of a product is most susceptible to microbial growth, the antimicrobial efficacy has been further assessed in analogy to the regulatory challenge test method (NF EN ISO11930) for an aqueous solution.

Thus, a solution of ***HPC1*** and/or Glyceryl caprylate in an amount as indicated in table 4 were prepared under sterile conditions. For this, it was solubilized in physiological serum with 0.85 wt.-% NaCl. The solutions were deposed in 96-deep well plates (1.6 ml/well). The wells were contaminated with *Aspergillus brasiliensis,* at 4.5*10⁴ cfu/ml. After the contamination each well was thoroughly mixed to ensure a homogeneous distribution of the microorganism.

Then each plate was incubated at 22°C for 24h. The counting of the (remaining) population was carried out 24h after contamination and reported in table 4. Composition 5 is a reference composition.

**Table 4 Antimicrobial action in the 24 hours screen test with Aspergillus brasiliensis.**

| | ***Ref.9*** | ***Ref. 10*** | ***Ref.11*** | **5** |
|---|---|---|---|---|
| ***HPC1*** [wt.%] | | 0.5 | | 0.5 |
| Glyceryl Caprylate [wt.%] | | | 0.01 | 0.01 |
| Colony counts after 24h [cfu/ml] | 45'000 | 700 | 340 | 100 |

The results of tables 2 and 3 and 4 clearly show the effect that these specific copolymers, i.e. the hyperbranched copolymers, increase the antimicrobial action of antimicrobial agents in a cosmetic composition. This effect is synergistic.

The results of tables 2 and 3 clearly show the effect that these specific copolymers, i.e. the hyperbranched copolymers, increase the antimicrobial action of antimicrobial agents in a cosmetic composition. This effect is synergistic.

## Claims

1. A cosmetic composition comprising
a) a hyperbranched copolymer **(HBC)** of the monomers
(i) dodecenyl succinic acid anhydride
(ii) diisopropanol amine
(iii) bis-dimethylaminopropyl amine
having terminal groups of the formula 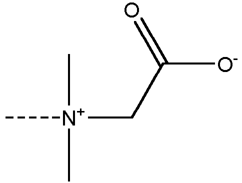
and having a molecular weight Mn of between 1200 and 4000 g/mol; and
b) an antimicrobial agent of the formula (I) 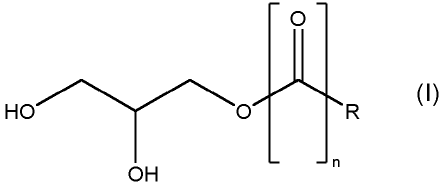
wherein R is a C₅-C₁₀ alkyl group or C₅-C₁₀ cyclo alkyl group, preferably a C₆-C₉ alkyl group or a C₆-C₉ cyclo alkyl group, more preferably a C₈ alkyl group or a C₆ cyclo alkyl group;
and wherein n is 0.

2. The cosmetic composition according to claim 1 **characterized in that** the hyperbranched copolymer **(HBC)** is prepared by the consecutive steps of
a1) polymerizing the monomers (i) and monomers (ii) and monomers (iii) to yield a polyesteramide having terminal dimethylamino groups of the formula 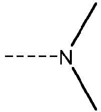
a2) quaternization of the dimethyl amino groups of the polyesteramide of step a1) by 2-chloroacetate, particularly by sodium 2-chloroacetate.

3. The cosmetic composition according to anyone of the preceding claims **characterized in that** the molar ratio of the monomers (i) to monomers (ii) is between 5 : 1 and 0.5 : 1, particularly between 4 : 1 and 1 : 1, preferably between 3 : 1 and 3 : 2.

4. The cosmetic composition according to anyone of the preceding claims **characterized in that** the molar ratio of the monomers (i) to monomers (iii) is between 5 : 1 and 0.5 : 1, particularly between 3 : 1 and 1 : 1, preferably between 2.5 : 1 and 1.1 : 1.

5. The cosmetic composition according to anyone of the preceding claims **characterized in that** the hyperbranched copolymer has a number average molecular weight Mₙ of between 1400 and 3000 g/mol, preferably between 2100 and 2400 g/mol.

6. The cosmetic composition according to anyone of the preceding claims **characterized in that** the hyperbranched copolymer is polyquaternium-110.

7. The cosmetic composition according to anyone of the preceding claims, **characterized in that** the antimicrobial agent is ethylhexylglycerin or hexylglycerin.

8. The cosmetic composition according to anyone of the preceding claims, **characterized in that** the amount of the hyperbranched copolymer **(HBC)** is between 0.005 and.5.0 % by weight, preferably between 0.05 and 5.0 % by weight, more preferably between 0.1 and 3.0 % by weight, most preferably between 0.5 and 2.5 % by weight, based on the total weight of the cosmetic composition.

9. The cosmetic composition according to anyone of the preceding claims, **characterized in that** the amount of the antimicrobial agent is 0.01 - 6.0 % by weight, preferably 0.05 - 5.0 % by weight, more preferably 0.1 - 3.0 % by weight, based on the total weight of the cosmetic composition.

10. The cosmetic composition according to anyone of the preceding claims, **characterized in that** the weight ratio of said hyperbranched copolymer **(HBC)** and antimicrobial agent is in the range of between 1:30 and 30:1, preferred between 1:20 and 20:1, more preferred between 1:10 and 10:1.

11. The cosmetic composition according to anyone of the preceding claims, **characterized in that** the cosmetic composition is a skin care composition.

12. The cosmetic composition according to anyone of the preceding claims, **characterized in that** the cosmetic composition is a hair care composition.

13. The cosmetic composition according to anyone of the preceding claims **characterized in that** the cosmetic composition is a shampoo or a hair conditioner.

14. The cosmetic composition according to anyone of the preceding claims **characterized in that** the cosmetic composition is a topical composition applied to the human skin, scalp and/ or hair.

15. Use of a hyperbranched copolymer **(HBC)** of the monomers
(i) dodecenyl succinic acid anhydride
(ii) diisopropanol amine
(iii) bis-dimethylaminopropyl amine
having terminal groups of the formula 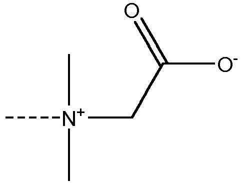
and having a molecular weight Mn of between 1200 and 4000 g/mol; to enhance synergistically the antimicrobial activity of an antimicrobial agent of the formula (I) 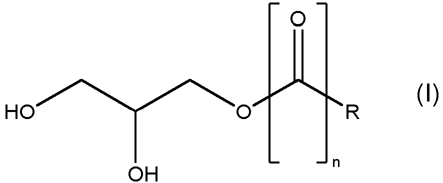
wherein R is a C₅-C₁₀ alkyl group or C₅-C₁₀ cyclo alkyl group, preferably a C₆-C₉ alkyl group or a C₆-C₉ cyclo alkyl group, more preferably a C₈ alkyl group or a C₆ cyclo alkyl group;
and wherein n is 0.

## Patentansprüche

1. Kosmetische Zusammensetzung, umfassend
a) ein hyperverzweigtes Copolymer (HBC) der Monomere
(i) Dodecenylbernsteinsäureanhydrid
(ii) Diisopropanolamin
(iii) Bis(dimethylaminopropyl)amin
mit endständigen Gruppen der Formel 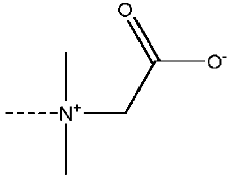
und mit einem Molekulargewicht Mₙ zwischen 1200 und 4000 g/mol; und
b) ein antimikrobielles Mittel der Formel (I) 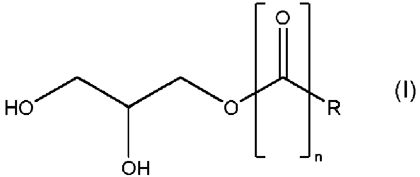
wobei R für eine C₅-C₁₀-Alkylgruppe oder C₅-C₁₀-Cycloalkylgruppe, vorzugsweise eine C₆-C₉-Alkylgruppe oder eine C₆-C₉-Cycloalkylgruppe, weiter bevorzugt eine C₈-Alkylgruppe oder eine C₆-Cycloalkylgruppe, steht;
und wobei n für 0 steht.

2. Kosmetische Zusammensetzung nach Anspruch 1, **dadurch gekennzeichnet, dass** das hyperverzweigte Copolymer (HBC) hergestellt wird durch die aufeinanderfolgenden Schritte 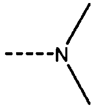
a1) Polymerisieren der Monomere (i) und Monomere (ii) und Monomere (iii) unter Erhalt eines Polyesteramids mit endständigen Dimethylaminogruppen der Formel 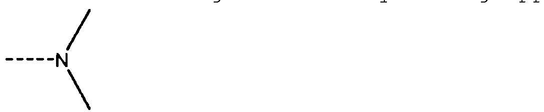
a2) Quaternisierung der Dimethylaminogruppen des Polyesteramids aus Schritt a1) mit 2-Chloracetat, insbesondere mit Natrium-2-chloracetat.

3. Kosmetische Zusammensetzung nach einem der vorhergehenden Ansprüche, **dadurch gekennzeichnet, dass** das Molverhältnis der Monomere (i) zu den Monomeren (ii) zwischen 5 : 1 und 0,5 : 1, besonders zwischen 4 : 1 und 1 : 1, bevorzugt zwischen 3 : 1 und 3 : 2.

4. Kosmetische Zusammensetzung nach einem der vorhergehenden Ansprüche, **dadurch gekennzeichnet, dass** das Molverhältnis der Monomere (i) zu den Monomeren (iii) zwischen 5 : 1 und 0,5 : 1, besonders zwischen 3 : 1 und 1 : 1, bevorzugt zwischen 2,5 : 1 und 1,1 : 1.

5. Kosmetische Zusammensetzung nach einem der vorhergehenden Ansprüche, **dadurch gekennzeichnet, dass** das hyperverzweigte Copolymer ein zahlenmittleres Molekulargewicht Mₙ zwischen 1400 und 3000 g/mol, bevorzugt zwischen 2100 und 2400 g/mol, aufweist.

6. Kosmetische Zusammensetzung nach einem der vorhergehenden Ansprüche, **dadurch gekennzeichnet, dass** es sich bei dem hyperverzweigten Copolymer um Polyquaternium-110 handelt.

7. Kosmetische Zusammensetzung nach einem der vorhergehenden Ansprüche, **dadurch gekennzeichnet, dass** es sich bei dem antimikrobiellen Mittel um Ethylhexylglycerin oder Hexylglycerin handelt.

8. Kosmetische Zusammensetzung nach einem der vorhergehenden Ansprüche, **dadurch gekennzeichnet, dass** die Menge des hyperverzweigten Copolymers (HBC) zwischen 0,005 und 5,0 Gew.-%, bevorzugt zwischen 0,05 und 5,0 Gew.-%, weiter bevorzugt zwischen 0,1 und 3,0 Gew.-%, ganz besonders bevorzugt zwischen 0,5 und 2,5 Gew.-%, bezogen auf das Gesamtgewicht der kosmetischen Zusammensetzung, liegt.

9. Kosmetische Zusammensetzung nach einem der vorhergehenden Ansprüche, **dadurch gekennzeichnet, dass** die Menge des antimikrobiellen Mittels 0,01 - 6,0 Gew.-%, bevorzugt 0,05 - 5,0 Gew.-%, weiter bevorzugt 0,1 - 3,0 Gew.-%, bezogen auf das Gesamtgewicht der kosmetischen Zusammensetzung, beträgt.

10. Kosmetische Zusammensetzung nach einem der vorhergehenden Ansprüche, **dadurch gekennzeichnet, dass** das Gewichtsverhältnis von hyperverzweigtem Copolymer (HBC) und antimikrobiellem Mittel im Bereich zwischen 1:30 und 30:1, bevorzugt zwischen 1:20 und 20:1, weiter bevorzugt zwischen 1:10 und 10:1, liegt.

11. Kosmetische Zusammensetzung nach einem der vorhergehenden Ansprüche, **dadurch gekennzeichnet, dass** es sich bei der kosmetischen Zusammensetzung um eine Hautpflegezusammensetzung handelt.

12. Kosmetische Zusammensetzung nach einem der vorhergehenden Ansprüche, **dadurch gekennzeichnet, dass** es sich bei der kosmetischen Zusammensetzung um eine Haarpflegezusammensetzung handelt.

13. Kosmetische Zusammensetzung nach einem der vorhergehenden Ansprüche, **dadurch gekennzeichnet, dass** es sich bei der kosmetischen Zusammensetzung um ein Shampoo oder einen Haarconditioner handelt.

14. Kosmetische Zusammensetzung nach einem der vorhergehenden Ansprüche, **dadurch gekennzeichnet, dass** es sich bei der kosmetischen Zusammensetzung um eine topische Zusammensetzung handelt, die auf die menschliche Haut, die menschliche Kopfhaut und/oder das menschliche Haar aufgebracht wird.

15. Verwendung eines hyperverzweigten Copolymers (HBC) der Monomere
(i) Dodecenylbernsteinsäureanhydrid
(ii) Diisopropanolamin
(iii) Bis(dimethylaminopropyl)amin
mit endständigen Gruppen der Formel 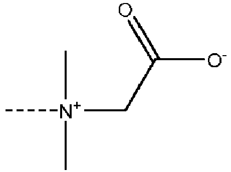
und mit einem Molekulargewicht Mₙ zwischen 1200 und 4000 g/mol;
zur synergistischen Verstärkung der antimikrobiellen Wirkung eines antimikrobiellen Mittels der Formel (I) 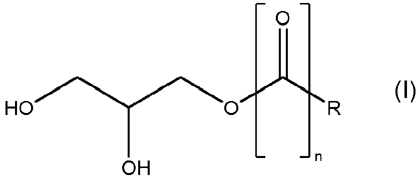
wobei R für eine C₅-C₁₀-Alkylgruppe oder C₅-C₁₀-Cycloalkylgruppe, vorzugsweise eine C₆-C₉-Alkylgruppe oder eine C₆-C₉-Cycloalkylgruppe, weiter bevorzugt eine C₈-Alkylgruppe oder eine C₆-Cycloalkylgruppe, steht;
und wobei n für 0 steht.

## Revendications

1. Composition cosmétique comprenant
a) un copolymère hyperramifié **(HBC)** des monomères
(i) anhydride d'acide dodécénylsuccinique
(ii) diisopropanolamine
(iii) bis-diméthylaminopropylamine
ayant des groupes terminaux de la formule 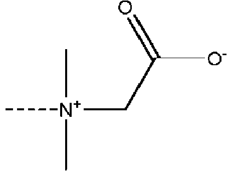
et ayant un poids moléculaire Mₙ compris entre 1 200 et 4 000 g/mole ; et
b) un agent antimicrobien de formule (I) 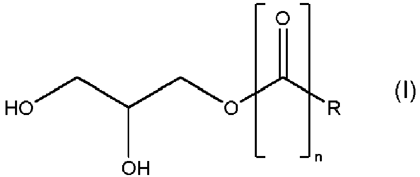
dans laquelle R est un groupe alkyle en C₅-C₁₀ ou un groupe cycloalkyle en C₅-C₁₀, de préférence un groupe alkyle en C₆-C₉ ou un groupe cycloalkyle en C₆-C₉, plus préférablement un groupe alkyle en C₈ ou un groupe cycloalkyle en C₆ ;
et dans laquelle n est 0.

2. Composition cosmétique selon la revendication 1, **caractérisée en ce que** le copolymère hyperramifié **(HBC)** est préparé par les étapes consécutives de
a1) polymérisation des monomères (i) et des monomères (ii) et des monomères (iii) pour donner un polyesteramide ayant des groupes diméthylamino terminaux de la formule 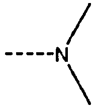
a2) quaternisation des groupes diméthylamino du polyesteramide de l'étape a1) par un 2-chloroacétate, en particulier par le 2-chloroacétate de sodium.

3. Composition cosmétique selon l'une quelconque des revendications précédentes, **caractérisée en ce que** le rapport molaire des monomères (i) sur les monomères (ii) est compris entre 5 : 1 et 0,5 : 1, particulièrement entre 4 : 1 et 1 : 1, de préférence entre 3 : 1 et 3 : 2.

4. Composition cosmétique selon l'une quelconque des revendications précédentes, **caractérisée en ce que** le rapport molaire des monomères (i) sur les monomères (iii) est compris entre 5 : 1 et 0,5 : 1, particulièrement entre 3 : 1 et 1 : 1, de préférence entre 2,5 : 1 et 1,1 : 1.

5. Composition cosmétique selon l'une quelconque des revendications précédentes, **caractérisée en ce que** le copolymère hyperramifié a un poids moléculaire moyen en nombre Mₙ compris entre 1 400 et 3 000 g/mole, de préférence entre 2 100 et 2 400 g/mole.

6. Composition cosmétique selon l'une quelconque des revendications précédentes, **caractérisée en ce que** le copolymère hyperramifié est le polyquaternium-110.

7. Composition cosmétique selon l'une quelconque des revendications précédentes, **caractérisée en ce que** l'agent antimicrobien est l'éthylhexylglycérine ou l'hexylglycérine.

8. Composition cosmétique selon l'une quelconque des revendications précédentes, **caractérisée en ce que** la quantité du copolymère hyperramifié **(HBC)** est comprise entre 0,005 et 5,0 % en poids, de préférence entre 0,05 et 5,0 % en poids, plus préférablement entre 0,1 et 3,0 % en poids, le plus préférablement entre 0,5 et 2,5 % en poids, par rapport au poids total de la composition cosmétique.

9. Composition cosmétique selon l'une quelconque des revendications précédentes, **caractérisée en ce que** la quantité de l'agent antimicrobien est de 0,01 à 6,0 % en poids, de préférence de 0,05 à 5,0 % en poids, plus préférablement de 0,1 à 3,0 % en poids, par rapport au poids total de la composition cosmétique.

10. Composition cosmétique selon l'une quelconque des revendications précédentes, **caractérisée en ce que** le rapport pondéral dudit copolymère hyperramifié **(HBC)** et de l'agent antimicrobien est dans la plage entre 1 : 30 et 30 : 1, de préférence entre 1 : 20 et 20 : 1, plus préférablement entre 1 : 10 et 10 : 1.

11. Composition cosmétique selon l'une quelconque des revendications précédentes, **caractérisée en ce que** la composition cosmétique est une composition de soin de la peau.

12. Composition cosmétique selon l'une quelconque des revendications précédentes, **caractérisée en ce que** la composition cosmétique est une composition de soin capillaire.

13. Composition cosmétique selon l'une quelconque des revendications précédentes, **caractérisée en ce que** la composition cosmétique est un shampooing ou un après-shampooing.

14. Composition cosmétique selon l'une quelconque des revendications précédentes, **caractérisée en ce que** la composition cosmétique est une composition topique appliquée sur la peau humaine, le cuir chevelu humain et/ou les cheveux humains.

15. Utilisation d'un copolymère hyperramifié **(HBC)** des monomères
(i) anhydride d'acide dodécénylsuccinique
(ii) diisopropanolamine
(iii) bis-diméthylaminopropylamine
ayant des groupes terminaux de la formule 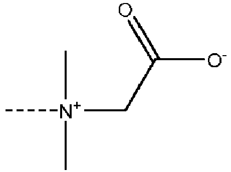
et ayant un poids moléculaire Mₙ compris entre 1 200 et 4 000 g/mole ;
pour améliorer de manière synergique l'activité antimicrobienne d'un agent antimicrobien de formule (I) 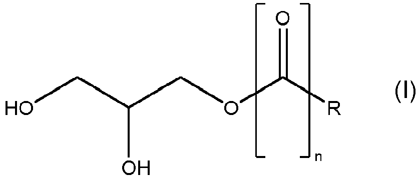
dans laquelle R est un groupe alkyle en C₅-C₁₀ ou un groupe cycloalkyle en C₅-C₁₀, de préférence un groupe alkyle en C₆-C₉ ou un groupe cycloalkyle en C₆-C₉, plus préférablement un groupe alkyle en C₈ ou un groupe cycloalkyle en C₆ ;
et dans laquelle n est 0.
